# EUROPEAN PATENT APPLICATION

(11) **EP 1 614 685 A1**
(43) Date of publication of application: **11.01.2006**
(21) Application number: 04356125.7
(22) Date of filing: 07.07.2004
(51) Int. Cl.: C07D 311/56

(54) **Process for preparing 2-chloro-6-Halogeno-3-alkyl-4H-chrom en-4-one or 2-chloro-3-alkyl-4H-chromen-4-one derivatives**

(71) Applicant: Bayer CropScience AG, 40789 Monheim (DE)
(72) Inventor: Himmler, Thomas, 51519 Odenthal (DE)
(74) Representative: Nowak, Alexander

(57) **Abstract**

The present invention relates to a process for preparing 2-chloro-6-halogeno-3-alkyl-4*H*-chromen-4-one or 2-chloro-3-alkyl-4*H*-chromen-4-one derivatives and to compounds that can be prepared with this process and to compounds useful for this process.

## Description

The present invention relates to a process for preparing 2-chloro-6-halogeno-3-alkyl-4*H*-chromen-4-one or 2-chloro-3-alkyl-4*H*-chromen-4-one derivatives and to compounds useful for this process and to compounds that can be prepared with this process.

The compounds that can be prepared with the process according to the invention are useful intermediate compounds for further preparing pesticides, in particular fungicide active substances. Certain of these substances have been disclosed in WO 97/13762 or WO 03/014103, for example.

There have been a number of processes disclosed for preparing 2,3-unsaturated ketones by dehydrohalogenation of a corresponding 2-halogen-substituted ketone by reaction with lithium chloride alone, an alkali carbonate alone, or a combination of an alkali carbonate with a lithium halogenide. For example, there is disclosed in R.P. Holysz, JACS **75** (1953) 4432-7 a process acoording to the following scheme: 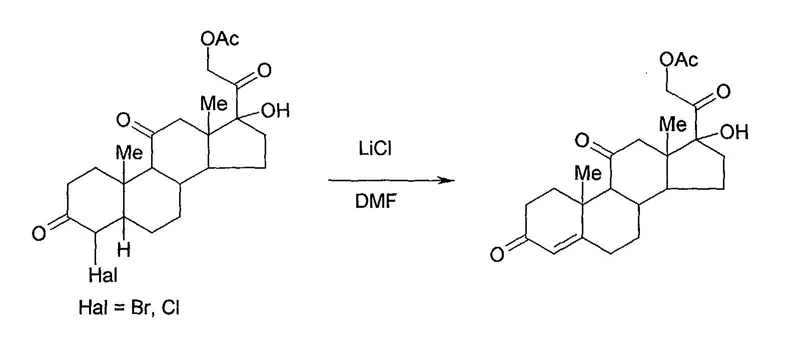 In this process, use of lithium carbonate is not mentioned and lithium chloride is used in high amounts that can be of up to 3 equivalents. Poor yields result from this process when used for preparing compounds of formula (I) according to the invention.
In T. Norin, Acta Chem. Scand. **17** (1963) 739-45, a process according to the following scheme is disclosed:

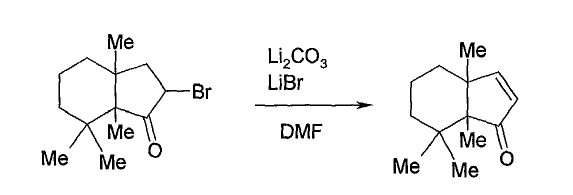

In this process, there is used a mixture of salts in N,N-dimethylformamide (DMF) to achieve the reaction, notably a mixture of lithium bromide and lithium carbonate in high amounts that can be up to 4.6 equivalents and 5.4 equivalents respectively. Again, poor yields result from this process when used for preparing compounds of formula (I) according to the invention.

Many of the known processes suffer from one or more drawbacks including low or poor yields, high relative amounts of reagent necessary to conduct the reaction or use of costly reagents.
The process of this invention surprisingly allows to eliminate these drawbacks.
For example, it has been possible with the present invention to use lower equivalent amounts of a base or of lithium chloride than in some prior known similar processes. With the process according to the invention, it has also surprisingly been possible to use potassium carbonate instead of the costly lithium carbonate as known in the prior art.
With such conditions, it has also been possible to obtain or maintain high yields.

Accordingly, the present invention provides a process for the preparation of a compound of formula (I) 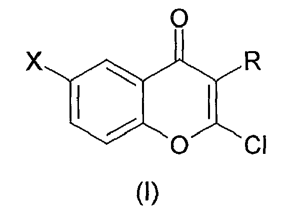
wherein X represents hydrogen or a halogen atom and R a substituted or non substituted C₁₋₁₂-alkyl ;
by reacting in a solvent a compound of formula (II) 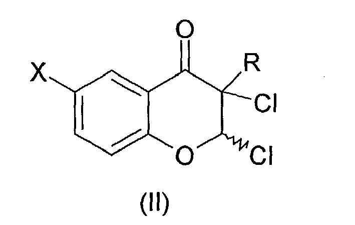
wherein X and R have the meanings as mentioned above, with a base MHCO₃ or M₂CO₃ in which M represents Li, Na K or Cs, in the presence of lithium chloride.

For the process of the invention X is preferably hydrogen or an iodine atom ; R is preferably a C₁₋₆-alkyl and more preferably a C₁₋₃-alkyl like methyl or propyl. A benzyl group is also a suitable R substituent.

Suitable solvents for carrying out the process according to the invention are dimethylformamide (DMF) or NMP (1-methyl-2-pyrrolidinone) or mixtures thereof. Preferred solvent is dimethylformamide.

Suitable bases for carrying out the process according to the invention are MHCO₃ or M₂CO₃ with M representing Li, Na, K or Cs. Preferred bases are M₂CO₃.
Amounts of base for carrying out the process according to the invention are advantageously in the range of from 0.5 to 4mol per mol of compound of formula (II) ; preferably this range is from 1 to 2mol.

The amount of lithium chloride for carrying out the process according to the invention is advantageously in the range of from 0.8 to 4mol per mol of compound of formula (II) ; preferably this range is from 1 to 2mol.

Suitable temperatures for carrying out the process according to the invention are in the range of from 100 to 200°C; preferably in the range of from 130 to 160°C.

Generally the reaction time for carrying out the process according to the invention is from 1 to 24h ; preferably from 2 to 6h.

In summary the course of the reaction according to the invention can be represented by the following scheme: 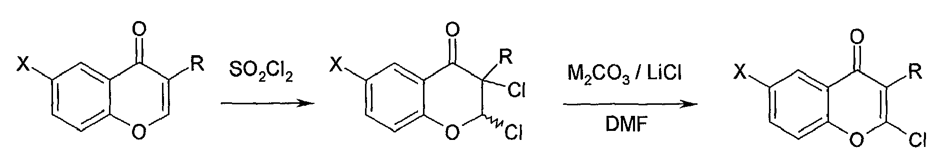 The present invention also relates to compounds formula (II) that are useful for the process according to the invention.
These compounds are of formula (II) wherein X represents hydrogen or a halogen atom preferably an iodine atom, and R represents a substituted or non substituted C₁₋₁₂-alkyl.
For the compounds of formula (II) according to the invention, R is preferably a C₁₋₆-alkyl and more preferably a C₁₋₃-alkyl like methyl or propyl. A benzyl group is also a suitable R substituent.
Examples of compounds of formula (II) according to the invention are:
- 2,3-dichloro-3-methyl-4*H*-chroman-4-one ;
- 2,3-dichloro-3-propyl-4*H*-chroman-4-one ;
- 2,3-dichloro-3-benzyl-4*H*-chroman-4-one ;
- 2,3,6-trichloro-3-methyl-4*H*-chroman-4-one;
- 2,3-dichloro-6-bromo-3-propyl-4*H*-chroman-4-one;
- 2,3-dichloro-6-iodo-3-methyl-4*H*-chroman-4-one ;
- 2,3-dichloro-6-iodo-3-propyl-4*H*-chroman-4-one ;
- 2,3-dichloro-6-iodo-3-benzyl-4*H*-chroman-4-one.

The compounds of formula (II) of the invention can prepared by known methods (for example: V.A. Zagorevskii et al., Chem. Heterocycl. Comp. (Engl. Transl.) **3** (1967) 624-6) herein-described in the examples. Where appropriate, these methods can be adapted by the skilled person. The 3-alkyl-chromones, used as starting materials to prepare the compounds of formular (II) of the invention, are known compounds or can be prepared by known methods.

The present invention further relates to compounds of formula (I) that can be prepared according to the process of the invention.
These compounds are of formula (I) wherein X represents hydrogen or a halogen atom with the exclusion of an iodine atom, and R represents a substituted or non substituted C₁₋₁₂-alkyl.
For the compounds of formula (I) according to the invention, R is preferably a C₁₋₆-alkyl and more preferably a C₁₋₃-alkyl like methyl or propyl. A benzyl group is also a suitable R substituent.
Examples of compounds of formula (I) according to the invention are:
- 2-chloro-3-methyl-4*H*-chromen-4-one ;
- 2-chloro-3-propyl-4*H*-chromen-4-one ;
- 2-chloro-3-benzyl-4*H*-chromen-4-one ;
- 2-chloro-6-chloro-3-methyl-4*H*-chromen-4-one ;
- 2-chloro-6-chloro-3-propyl-4*H*-chromen-4-one ;
- 2-chloro-6-bromo-3-propyl-4*H*-chromen-4-one.

The following examples are given to illustrate the various aspects of the present invention without any limitation.

### Examples of the process according to the invention to prepare 2-chloro-3-alkyl-4H-chromen-4-one:

### 2-chloro-3-methyl-4H-chromen-4-one (with Li₂CO₃ and LiCl in DMF)

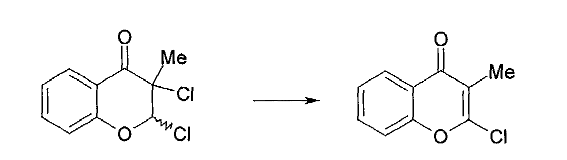

A mixture of 11.56g of 2,3-dichloro-3-methyl-4*H*-chromen-4-one, 4.95g Li₂CO₃ and 2.84g LiCl in 150ml DMF is refluxed for 2 hours. The reaction mixture is cooled to room temperature, filtered, and the filtrate is concentrated under reduced pressure. The resulting residue is redissolved in t-butyl-methyl-ether and the solution is washed with water and brine. After drying over Na₂SO₄ the solvent is evaporated, giving 9.2g of solid which, according to HPLC analysis, has a purity of 83.7% (79.1% yield).
¹H-n.m.r. (400 MHz, CDCl₃): δ = 2,04 (s, 3H), 7.51-7.54 (m, 1H), 7.64-7.66 (m, 1 H), 7.80-7.85 (m, 1 H), 8,02-8,05 (1 H) ppm.

### 2-chloro-3-methyl-4H-chromen-4-one (with K₂CO₃ and LiCl in DMF)

A mixture of 0.347g 2,3-dichloro-3-methyl-4*H*-chromen-4-one, 0.276g K₂CO₃ and 0.085g LiCl in 5ml DMF is refluxed for 2 hours. After workup like described above a yield of 79% of theory results.

### 2-chloro-3-methyl-4H-chromen-4-one (with Li₂CO₃ and LiCl in NMP)

A mixture of 0.347g 2,3-dichloro-3-methyl-4*H*-chromen-4-one, 0.148g Li₂CO₃ and 0.085g LiCl in 5ml NMP is heated to 155°C for 2 hours. After work-up like described above a yield of 47% of theory results.

### Comparative example for preparing 2-chloro-3-methyl-4H-chromen-4-one (with K₂CO₃ and without LiCl in DMF)

A mixture of 0.347g of 2,3-dichloro-3-methyl-4*H*-chromen-4-one and 0.276g K₂CO₃ in 5ml DMF is refluxed for 6 hours. After work-up like described above a yield of only 31% of theory results (the primary product is the 3-methyl-4*H*-chromen-4-one).

### Comparative example for preparing 2-chloro-3-methyl-4H-chromen-4-one (with Li₂CO₃ and LiBr in DMF)

A mixture of 0.347g 2,3-dichloro-3-methyl-4H-chromen-4-one, 0.222g Li₂CO₃ and 0.261g LiBr in 5ml DMF is refluxed for 2 hours. After work-up like described above a yield of only 27% of theory results (the primary product is the 3-methyl-4*H*-chromen-4-one).

### Comparative example for preparing 2-chloro-3-methyl-4H-chromen-4-one (with K₂CO₃ and LiCl in DMA)

A mixture of 0.347g 2,3-dichloro-3-methyl-4*H*-chroman-4-one, 0.276g K₂CO₃ and 0,085g LiCl in 5ml N,N-dimethylacetamide (DMA) is heated to 150°C for 4 hours. After work-up like described above a yield of only 17% of theory results.

### Comparative example for preparing 2-chloro-3-methyl-4H-chromen-4-one (with LiCl only)

A mixture of 0.347g of 2,3-dichloro-3-methyl-4*H*-chroman-4-one and 0,085g LiCl in 5ml DMF is refluxed for 2 hours. After work-up like described above a yield of only 18% of theory results.

### 2-chloro-6-iodo-3-propyl-4H-chromen-4-one

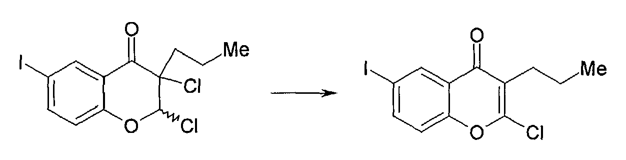

A mixture of 3.85g of 2,3-dichloro-6-iodo-3-propyl-4*H*-chroman-4-one, 1,03g Li₂CO₃ and 0.59g LiCl in 25ml DMF is stirred 4 hours at 150°C. The reaction mixture is cooled to room temperature, filtered, and the filtrate is concentrated under reduced pressure. The resulting residue is redissolved in dichloromethane and the solution is washed with water and brine. After drying over Na₂SO₄ the solvent is evaporated. This gives 3.1g of a brown oil which, according to GC analysis, has a purity of 84.4% (75.2% yield).
Crystallisation from isopropanol gives the product with 93.6% purity according to GC.
¹H-n.m.r. (400 MHz, d-DMSO): δ = 0.90-0.94 (t, 3H), 1.45-1.54 (m, 2H), 2.46-2.5 (m, 2H) 7.46-7.49 (dd, J=8.8 and 2.2 Hz; 1 H), 8,07-8.10 (dd, J= 8.8 and 2.2Hz, 1 H=, 8.24-8.25 (d, J= 2.2Hz, 1 H) ppm.

### 2-chloro-6-bromo-3-propyl-4H-chromen-4-one

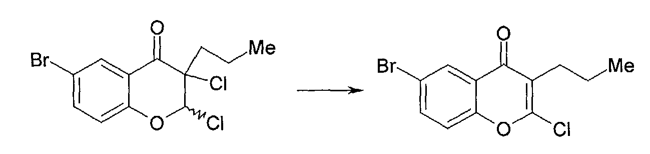

A mixture of 13.35g of 2,3-dichloro-6-bromo-3-propyl-4*H*-chroman-4-one, 4,09g Li₂CO₃ and 2.35g LiCl in 100ml DMF is stirred 4 hours at 150°C. The reaction mixture is cooled to room temperature, filtered, and the filtrate is concentrated under reduced pressure. The resulting residue is redissolved in dichloromethane and the solution is washed with water and brine. After drying over Na₂SO₄ the solvent is evaporated. This gives 10g of a brown solid which, according to GC analysis, contents 58.9% of the desired product (49.4% yield).
Crystallisation from isopropanol gives the product with 86.6% purity according to GC.

### 2,6-dichloro-3-methyl-4H-chromen-4-one

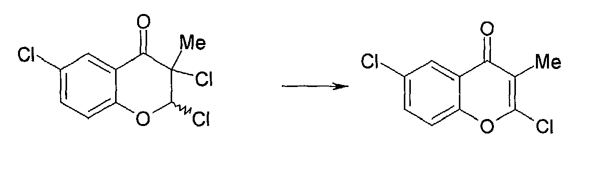

A mixture of 2.12g of 2,3,6-trichloro-3-methyl-4H-chroman-4-one, 1.48g K₂CO₃ and 0.45g LiCl in 25ml DMF is stirred for 2 hours at 150°C. The reaction mixture is cooled to room temperature, filtered, and the filtrate is concentrated under reduced pressure.
The resulting residue is redissolved in dichloromethane and the solution is washed with water and brine. After drying over Na₂SO₄, the solvent is evaporated, giving 1.8g of a solid which, according to GC analysis, contents 74.7% of the desired product (73.4% yield).

### 2-chloro-3-benzyl-4H-chromen-4-one

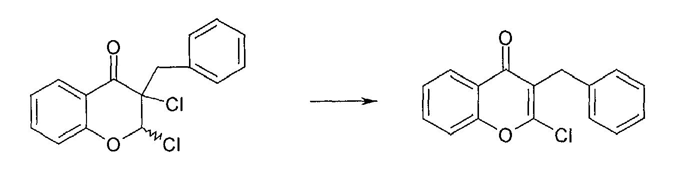

A mixture of 2.46g of 2,3-dichloro-3-benzyl-4H-chroman-4-one, 1.47g K₂CO₃ and 0.45g LiCl in 25ml DMF is stirred for 2 hours at 150°C. The reaction mixture is cooled to room temperature, filtered, and the filtrate is concentrated under reduced pressure. The resulting residue is redissolved in dichloromethane and the solution is washed with water and brine. After drying over Na₂SO₄ the solvent is evaporated. This gives 2.4g of an orange oil which, according to GC analysis, contents 46.3% of the desired product (51.3% yield).
Chromatography on silica (eluent: hexane / ethyl acetate 4 : 1) gives an orange solid with a purity of nearly 80%.
¹H-n.m.r. (400 MHz, d-DMSO): δ = 3.90 (s, 2H), 7.18-7.31 (m, 5H), 7.52-7.56 (m, 1H),, 7.66-7.68 (m, 1 H), 7.82-7.86 (m, 1 H), 8,05-8,07 (m, 1 H) ppm.

### Examples of 2,3-dichloro-3-alkyl-4H-chroman-4-one and 2,3-dichloro-6-halogeno-3-alkyl-4H-chroman-4-one:

(products of the first step of the process according to the invention):

### 2,3-dichloro-3-methyl-4H-chroman-4-one

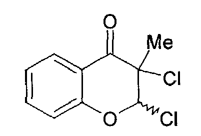

At room temperature to 24,03g of 3-methyl-chromone 25.3g of sulfuryl chloride are added dropwise. After additional stirring for 1 hour at room temperature 50ml of dichloromethane are added. The reaction mixture is stirred for 2 hours at 35°C. Then the mixture is washed with water at room temperature. The organic phase is dried over Na₂SO₄ and the solvent distilled off under reduced pressure. This gives 33.4g of a pale yellow solid which, according to HPLC analysis, consists of two isomers of the desired product in a ratio of 76 : 24. The purity is 95.4% (91.9% yield).
The main isomer can be obtained in high purity of 98% by stirring the mixture with petroleum ether , filtration and drying.
¹H-n.m.r. (600 MHz, CDCl₃): δ = 1.89 (s, 3H), 6.39 (s, 1H), 7,08-7,09 (d, 8.3Hz, 1H), 7.21-7.26 (m, 1H), 7.60-7.63 (m, 1H), 8,01-8,03 (dd, 7.8 and 1.5Hz, 1H) ppm.
Melting point: 99.5-100.5°C

### 2,3-dichloro-6-iodo-3-propyl-4H-chroman-4-one

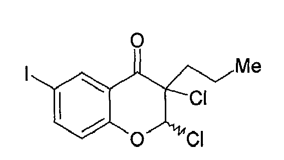

A mixture of 1.57g of 6-lodo-3-propyl-4*H*-chromen-4-one and 1.68g of sulfuryl chloride in 2ml of dichloromethane is stirred at room temperature for 48 hours. After concentration under reduced pressure 1.9g of a yellow oil result which, according to HPLC analysis, consist of two isomers of the desired product in a ratio of 84 : 16. The purity is 87.5% (86.4% yield).

### 2,3-dichloro-6-bromo-3-propyl-4H-chroman-4-one

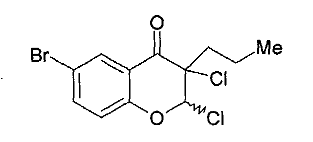

A mixture of 10.7g of 6-bromo-3-propyl-4*H*-chromen-4-one and 10.1g of sulfuryl chloride in 5ml of 1.2-dichloroethane is stirred at 45°C for about 4 hours and after that at 70°C for 1 hour. Concentration under reduced pressure gives 15g of a solid which, according to HPLC analysis, consists of two isomers of the desired product in a ratio of 91 : 9. The purity is 89.1 % (98.8% yield).

### 2,3,6-trichloro-3-methyl-4H-chroman-4-one

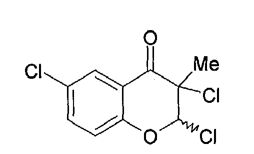

A mixture of 7.78g of 6-chloro-3-methyl-4*H*-chromen-4-one and 10.1g of sulfuryl chloride in 5ml of 1.2-dichloroethane is stirred at 45°C for about 4 hours and after that at 70°C for 1 hour. After that time additional amounts of 3ml of sulfuryl chloride and 5ml of 1.2-dichloroethane are added and the mixture is heated to 70°C for another 4 hours. After concentration under reduced pressure 13.6g of a yellow solid result which, according to HPLC analysis, consists of two isomers of the desired product in a ratio of 87 : 13. The purity is 47% (60.2% yield).
The primary isomer of the product can be obtained as a pure compound (93.4% according to GC analysis) by crystallisation from isopropanol.

### 2,3-dichloro-3-benzyl-4H-chroman-4-one

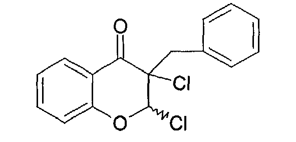

A mixture of 9.45g of 3-benzyl-4*H*-chromen-4-one and 10g of sulfuryl chloride is stirred at 45°C for 4 hours. After cooling to room temperature hexane is added, the solid is collected, washed with hexane and dried. This results in 9.8g of a solid which, according to GC analysis, has a purity of 91.6% (73.1% yield).

## Claims

1. A process for the preparation of a compound of formula (I) 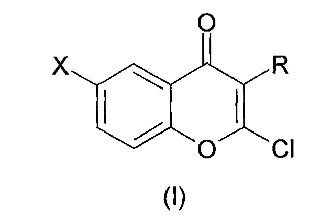
wherein X represents hydrogen or a halogen atom and R a substituted or non substituted C₁₋₁₂-alkyl ;
by reacting in a solvent a compound of formula (II) 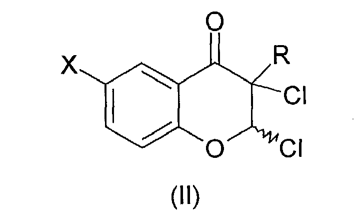
wherein X and R have the meanings as mentioned above,
with a base MHCO₃ or M₂CO₃ in which M represents Li, Na K or Cs, in the presence of lithium chloride.

2. A process according to claim 1 wherein X is hydrogen or an iodine atom.

3. A process according to claim 1 or 2 wherein R is a C₁₋₆-alkyl.

4. A process according to claim 3 wherein R is methyl, propyl or benzyl.

5. A process according to claim 1 to 4 wherein the solvent is dimethylformamide (DMF).

6. A process according to claim 1 to 5 wherein the base is M₂CO₃ with M representing Li, Na, K or Cs.

7. A process according to claim 1 to 6 wherein the amount of base is in the range of from 1 to 2mol.

8. A process according to claim 1 to 7 wherein the amount of lithium chloride is in the range of from 1 to 2mol of compound of formula (II).

9. A compound of formula (I) 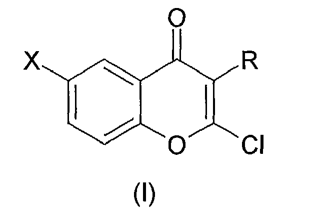
wherein X represents hydrogen or a halogen atom with the exclusion of an iodine atom, and R represents a substituted or non substituted C₁₋₁₂-alkyl.

10. A compound according to claim 9 wherein R is a C₁₋₆-alkyl or a benzyl group.

11. A compound according to claim 10 wherein R is a C₁₋₃-alkyl.

12. A compound according to claim 11 wherein R is methyl or propyl.

13. A compound according to claims 9 to 12 which is:
- 2-chloro-3-methyl-4*H*-chromen-4-one ;
- 2-chloro-3-propyl-4*H*-chromen-4-one ;
- 2-chloro-3-benzyl-4*H*-chromen-4-one ;
- 2-chloro-6-chloro-3-methyl-4*H*-chromen-4-one ;
- 2-chloro-6-chloro-3-propyl-4*H*-chromen-4-one ;
- 2-chloro-6-bromo-3-propyl-4*H*-chromen-4-one.

14. A compound of formula (II) 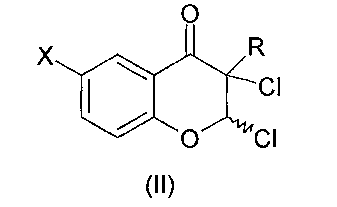
wherein X represents hydrogen or a halogen atom and R represents a substituted or non substituted C₁₋₁₂-alkyl.

15. A compound according to claim 14 wherein X represents an iodine atom and R represents a C₁₋₆-alkyl.

16. A compound according to claim 14 or 15 wherein R is methyl, propyl or benzyl.

17. A compound according to claim 14 to 16 which is:
- 2,3-dichloro-3-methyl-4*H*-chroman-4-one ;
- 2,3-dichloro-3-propyl-4*H*-chroman-4-one ;
- 2,3-dichloro-3-benzyl-4*H*-chroman-4-one ;
- 2,3-dichloro-6-iodo-3-methyl-4*H*-chroman-4-one ;
- 2,3-dichloro-6-iodo-3-propyl-4*H*-chroman-4-one ;
- 2,3-dichloro-6-iodo-3-benzyl-4*H*-chroman-4-one.
